# EUROPÄISCHE PATENTSCHRIFT

(11) **EP 1 212 285 B1**
(45) Veröffentlichungstag und Bekanntmachung des Hinweises auf die Patenterteilung: **02.02.2005**
(21) Anmeldenummer: 00965995.4
(22) Anmeldetag: 13.09.2000
(51) Int. Cl.: C07C 67/343, C07C 69/732, C07C 69/675

(54) **VERFAHREN ZUR HERSTELLUNG VON 2-HYDROXYCARBONSÄUREESTERN**
METHOD OF PRODUCING 2-HYDROXYCARBOXYLIC ACID ESTERS
PROCEDE DE PREPARATION D'ESTERS D'ACIDE 2-HYDROXY CARBOXYLIQUE

(30) Priorität: 14.09.1999 DE 19944049
(43) Veröffentlichungstag der Anmeldung: 12.06.2002
(73) Patentinhaber: Abbott GmbH & Co. KG, 65205 Wiesbaden (DE)
(72) Erfinder: KNOPP, Monika, 4322 Mumpf (CH); JANSEN, Rolf, 68159 Mannheim (DE)
(74) Vertreter: Kinzebach, Werner, Dr.
(86) Internationale Anmeldenummer: PCT/EP2000/008952
(87) Internationale Veröffentlichungsnummer: WO 2001/019774

(56) Entgegenhaltungen:
- G.BOIREAU ET AL.: "The Ate Complexes of Aluminium" TETRAHEDRON., Bd. 36, Nr. 20-21, 1980, Seiten 3061-3070, XP002156619 ELSEVIER SCIENCE PUBLISHERS, AMSTERDAM., NL ISSN: 0040-4020
- "Stereoselective Epoxidation of Acyclic Olefinic Carboxylic Acids via Iodolactonization" JOURNAL OF THE AMERICAN CHEMICAL SOCIETY., Bd. 100, Nr. 12, 7. Juni 1978 (1978-06-07), Seiten 3950-3951, XP002156620 AMERICAN CHEMICAL SOCIETY, WASHINGTON, DC., US ISSN: 0002-7863

## Beschreibung

Die vorliegende Erfindung betrifft ein Verfahren zur Herstellung von 2-Hydroxycarbonsäureestern mit quartärem β-Kohlenstoffatom.

Endothelinrezeptorantagonisten sind neue Wirkstoffe zur Behandlung verschiedener Herz-Kreislauf-Erkrankungen. In der WO 97/38981 sind verschiedene Endothelinrezeptorantagonisten beschrieben, z. B. (S)-3,3-Diphenyl-2-(4,6-dimethylpyrimid-2-yloxy)buttersäure. Diese Verbindung wird gemäß der Beschreibung in der WO 97/38981 aus (S)-2-Hydroxy-3,3-diphenylbuttersäure durch Umsetzung mit 4,6-Dimethylpyrimidin-3-ylsulfon erhalten. Die 2-Hydroxy-3,3-diphenylbuttersäure wird ihrerseits durch Reduktion von 2,2-Diphenylpropionsäurenitril zum Aldehyd, und dessen Umsetzung zum Cyanhydrin erhalten, das dann sauer hydrolysiert wird. Dieses Verfahren weist jedoch mehrere Nachteile auf. Bei der Cyanhydrinsynthese ist die aus sicherheitstechnischen und gesundheitlichen Aspekten nicht unbedenkliche Handhabung von Blausäure nötig. Weiterhin stellt 2,2-Diphenylpropionitril ein vergleichsweise teures Ausgangsmaterial dar, und die auf diesem Weg erhaltene Gesamtausbeute ist nicht zufriedenstellend.

Der vorliegenden Erfindung liegt daher die Aufgabe zugrunde, ein alternatives Verfahren zur Herstellung von 2-Hydroxycarbonsäuren mit quartärem β-Kohlenstoffatom bzw. deren Estern anzugeben.

Fukumasa, et al., THL 1991, 32, 1059-1062, beschreibt die Umsetzung einfach substituierter Epoxide mit Trimethylaluminium.

Danishewsky, S. et al., J. Org. Chem. 1976, 41, 1669-1671, beschreibt die Verwendung funktionalisierter Alane zur Umwandlung von Epoxiden in trans-anellierte γ-Lactone.

Kuran, W. et al., J. Organomet. Chem 1974, 73, 187-193, beschreibt die Umsetzung von Methylaluminiumverbindungen mit Propylenoxid.

Visnick, M. et al. Synthesis 1983, 284-287, beschreibt die Addition von t-Butoxycarbonylmethyldiethylalan an 1-Alkyliden-2,3-epoxy-3-methylcyclohexanen.

Pfaltz, A. et al., Angew. Chem. Int. Ed. Engl. 1982, 21, 71, berichten von der regioselektiven Ringöffnung von α- und β-Alkoxyepoxiden mit Trimethylaluminium in Gegenwart katalytischer Mengen von Butyllithium oder Lithiummethoxid.

Alexakis, A. et al., Tetrahedron, 1989, 45, 6197-6202 beschreiben die Bortrifluorid-unterstützte Ringöffnung von Epoxiden durch Lithiumalkenylaluminat-Reagenzien. Die verwendeten Epoxide waren Cyclohexenoxid und n-Butylepoxid.

Gorzynski-Smith, J., Synthesis, 1984, 634, weist allgemein auf die Möglichkeit der Umsetzung von Epoxiden mit Organoaluminiumverbindungen hin. Einfache Trialkylalane sollen von eingeschränkter Nützlichkeit sein, weil die Umsetzung durch unerwünschte Nebenreaktionen, wie die Reduktion des Epoxids, begleitet ist.

Miyashita, M. et al., J. Org. Chem. 1991, 56, 6483-6485, beschreibt die stereospezifische Methylierung von γ,δ-Epoxyacrylaten durch Trimethylaluminium in Gegenwart von Wasser. Miyashita, M. et al., Tetrahedron Asym. 1993, 4, 1573-1578, beschreibt die Anwendung der Epoxidringöffnung mit Trimethylaluminium in Gegenwart von Wasser bei der Synthese von (-)-Serricornin. Miyashita, M. et al., Chem. Soc., Chem. Commun. 1996, 9, 1027-1028, beschreibt die Anwendung der Epoxidringöffnung mit Trimethylaluminium in Gegenwart von Wasser bei der Synthese des Ansakettensegments von Streptovaricin U.

Aus Poon, T. et al., Synthesis 1998, 832, ist folgende Umsetzung bekannt:

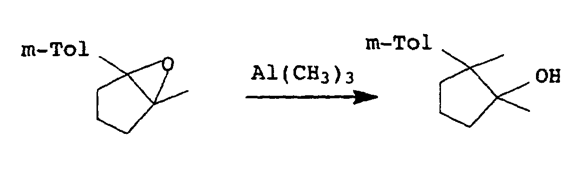

Die vorstehenden Literaturstellen offenbaren keine Umsetzungen, bei denen der Epoxidring eine Estergruppe trägt.

Neukom, C. et al., J. Am. Chem. Soc. 1986, 108, 5559-5568, beschreiben die Umsetzung von trans-Ethyl-2,3-epoxybutyrat mit Diethylpropynylalan. Bartlett, A. et al., J. Org. Chem. 1982, 47, 3941-3945, beschreiben die Umsetzung von Ethyl-trans-2,3-epoxybutanoat mit Diethyl-trimethylsilylethylalan. Die Umsetzung zu den α-Hydroxyestern mit tertiärem β-Kohlenstoffatom gelingt nur in mittleren Ausbeuten.

Erfindungsgemäß wird die gestellte Aufgabe durch ein Verfahren zur Herstellung von 2-Hydroxycarbonsäureestern der Formel I 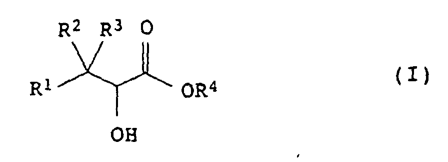 gelöst, worin
- R¹ und R²: unabhängig voneinander für C₁-C₂₀-Alkyl, C₃-C₈-Cycloalkyl, C₂-C₂₀-Alkenyl, C₂-C₂₀-Alkinyl, C₆-C₁₀-Aryl oder C₇-C₁₄-Aralkyl oder C₇-C₂₀-Alkaryl stehen, oder R¹ und R² gemeinsam mit dem Kohlenstoffatom, an das sie gebunden sind, einen 5- bis 8-gliedrigen Ring bilden;
- R³: für C₁-C₂₀-Alkyl, C₂-C₂₀-Alkenyl oder C₂-C₂₀-Alkinyl steht;
- R⁴: für C₁-C₂₀-Alkyl, C₃-C₈-Cycloalkyl, C₂-C₂₀-Alkenyl, C₂-C₂₀-Alkinyl, C₆-C₁₀-Aryl, C₇-C₁₄-Aralkyl oder C₇-C₂₀-Alkaryl steht;
bei dem man einen Glycidester der Formel II 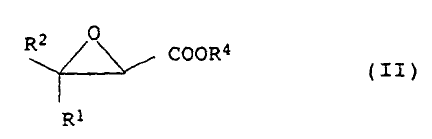 worin R¹, R² und R⁴ die angegebene Bedeutung haben,
mit einem aluminiumorganischen Reagenz der Formel III 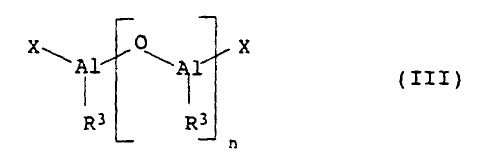 umsetzt, worin R³ die angegebenen Bedeutung hat, X jeweils unabhängig für die für R³ angegebenen Bedeutungen, Halogen oder C₁-C₄-Alkoxy steht und n für 0 bis 10 steht.

Als C₁-C₂₀-Alkyl kommen geradkettige und verzweigte Alkylgruppen, vorzugsweise C₁-C₈-Alkyl, wie Methyl, Ethyl, Propyl, n-Butyl, Isobutyl, t-Butyl, Pentyl, in Betracht.

Als C₃-C₈-Cycloalkyl kommt Cyclopropyl, Cyclobutyl, Cyclopentyl oder Cyclohexyl in Betracht.

Als C₂-C₂₀-Alkenyl kommt vorzugsweise C₁-C₈-Alkenyl, wie Vinyl, Allyl, 1-Hexenyl in Betracht.

Als C₂-C₂₀-Alkinyl kommt vorzugsweise C₁-C₈-Alkinyl, z. B. Ethinyl oder Propinyl, in Betracht.

Als C₆-C₁₀-Aryl kommt insbesondere Phenyl oder Naphthyl in Betracht.

Als C₇-C₁₄-Aralkyl kommt z. B. Benzyl oder Phenethyl in Betracht.

Als C₇-C₂₀-Alkylaryl kommt z. B. 2-, 3-, 4-Methylphenyl, 2,3-, 2,4-, 2,5-, 2,6-, 3,4-, 3,5-Dimethylphenyl oder 2,4,6-Trimethylphenyl in Betracht.

Vorzugsweise steht wenigstens einer der Reste R¹ und R² für Aryl, Aralkyl oder Alkylaryl, Cycloalkyl oder verzweigtes Alkyl, insbesondere Alkyl mit einer Verzweigung in 1- oder 2-Position, wie Isopropyl, t-Butyl, Isobutyl.

Besonders bevorzugt stehen R¹ und R² gleichzeitig für Phenyl.

R¹, R² und R³ können 1, 2, 3, 4 oder 5 Substituenten tragen, die die erfindungsgemäße Umsetzung nicht beeinträchtigen, wie z. B. C₁-C₆-Alkyl, C₁-C₆-Alkoxy-, Di(C₁-C₆-alkyl)amino-, Nitro-, Ester-(z. B. CO₂R⁵, wobei R⁵ die für R⁴ angegebenen Bedeutungen einnehmen kann), Amid-, Sulfonamid-, Silyl- oder Nitrilgruppen.

Die Glycidester der Formel II sind z. B. mittels Darzens-Glycidester-Synthese aus entsprechenden Ketonen durch Umsetzung mit Chloressigsäureestern und Base erhältlich. Sie sind ferner z. B. durch Epoxidierung geeignet substituierter Zimtsäureester erhältlich. Sie können am α-Kohlenstoff (S)- oder (R)-Konfiguration aufweisen und als reine oder angereicherte Enantiomere oder als Racemat vorliegen. Für den Fall, dass R¹ ≠ R², können auch am β-Kohlenstoff beide Konfigurationen vorliegen.

R³ steht vorzugsweise für Methyl, Ethyl, n-Butyl, besonders bevorzugt für Methyl. R³ kann ferner z. B. für AlkOCO-CH₂-, AlkO-C≡C-, Alk-C≡C-, Alk-CH=CH-, Alk-CH=CH-CH₂⁻ stehen, worin Alk für C₁-C₄-Alkyl steht.

In der Formel III steht X für die für R³ angegebenen Bedeutungen, insbesondere für C₁-C₄-Alkyl; Halogen, wie Fluor, Chlor oder Brom; oder C₁-C₄-Alkoxy.

Der Index n ist vorzugsweise 0. Besonders bevorzugte aluminiumorganische Reagenzien sind Trimethylaluminium, Triethylaluminium und Tributylaluminium, wobei Trimethylaluminium am meisten bevorzugt ist. Andere geeignete Reagenzien sind z. B. AlkOCOCH₂Al(C₂H₅)₂, Alk-CH=CH-Al(C₂H₅)₂.

Aluminiumorganische Reagenzien, in denen n ≠ 0 ist, sind unter der Bezeichnung Alumoxane bekannt und durch kontrollierte Umsetzung von Aluminiumoragnylen mit Wasser erhältlich (vgl. z. B. die DE-A-37 31 665).

Die erfindungsgemäße Umsetzung des Glycidesters der Formel II mit dem aluminiumorganischen Reagenz der Formel III erfolgt vorzugsweise bei einer Temperatur von weniger als 20 °C, insbesondere bei einer Temperatur im Bereich von -10 bis +10 °C.

Die erfindungsgemäße Umsetzung wird mit Vorteil in einem unpolarem Lösungsmittel, vorzugsweise einem aliphatischen oder aromatischen Kohlenwasserstoff oder Gemisch von aliphatischen und/oder aromatischen Kohlenwasserstoffen, wie Hexan, Heptan, Cyclohexan, Benzol, Toluol oder Xylol, durchgeführt.

Die Reaktionsdauer beträgt im Allgemeinen 0,5 bis 2 h. Nach beendeter Umsetzung wird das Reaktionsgemisch in der Regel sauerwässrig aufgearbeitet; der 2-Hydroxycarbonsäureester der Formel I wird nach üblichen Verfahren isoliert. Der Ester kann gewünschtenfalls in die zugrunde liegende Säure umgewandelt werden.

Vorzugsweise wird bei der erfindungsgemäßen Umsetzung des aluminiumorganischen Reagenz der Formel III im Überschuss verwendet. Besonders bevorzugt liegt das molare Verhältnis des aluminiumorganischen Reagenzes der Formel III zum Glycidester der Formel II im Bereich von 1,3 bis 1,5. Höhere Überschüsse an aluminiumorganischen Reagenz der Formel III können zu einer Verschlechterung der Regioselektivität führen.

Die erfindungsgemäße Umsetzung kann sowohl durch Zugabe des aluminiumorganischen Reagenzes zu einer Lösung des Glycidesters, vorzugsweise in einem unpolarem Lösungsmittel, erfolgen. Andererseits kann der Glycidester, vorzugsweise als Lösung in einem unpolaren Lösungsmittel, zu einer Lösung des aluminiumorganischen Reagenzes gegeben werden. Besonders bewährt hat sich eine Reaktionsführung, bei der eine Lösung des Glycidesters in Toluol zu einer Lösung des aluminiumorganischen Reagenzes in Heptan oder Cyclohexan gegeben wird.

Bei der erfindungsgemäßen Umsetzung erfolgt die Einführung des Restes R³ weitgehend regioselektiv in die β-Position zur Estergruppe. Die Regioselektivität beträgt im Allgemeinen mehr als 80 %, vorzugsweise mehr als 90 %. Die Umsetzung erfolgt überwiegend unter Erhalt der Konfiguration am Kohlenstoffatom, das die Estergruppe trägt. Bei Einsatz von Glycidester mit definierter Stereochemie können daher im Wesentlichen reine Enantiomere erhalten werden.

Die Umsetzung des Glycidesters mit dem aluminiumorganischen Reagenz gelingt überraschenderweise im Allgemeinen ohne Zusatz weiterer aktivierender Reagenzien, wie tertiären Aminen, Lithiumalkoxiden oder Lithiumalkylen, sogar bei Einsatz sterisch gehinderter Glycidester als Ausgangsmaterial.

Überraschenderweise werden außerdem bei der Umsetzung des Glycidesters mit dem Alkylaluminiumreagenz keine Nebenreaktionen, wie z. B. Eliminierung zu α,β-ungesättigten Estern oder Pinakol-Umlagerungen, beobachtet. Dies überrascht insbesondere, da z. B. Trialkylaluminium bekanntermaßen Pinakol-Umlagerungen katalysiert.

Die Erfindung wird nun durch die folgenden Beispiele näher veranschaulicht.

### Beispiel 1:

### Epoxidöffnung in Heptan bei -10 °C mit 1,3 Äquivalenten Trimethylaluminium (TMA)

190 g (747 mmol) 2,3-Epoxy-3,3-diphenylpropionsäuremethylester wurden in 80 ml Toluol gelöst und zu 920 ml Heptan gegeben. Bei -10 °C wurden 486 ml (971 mmol) 2M Trimethylaluminiumlösung in Toluol zugegeben. Nach beendeter Zugabe lag eine schwachgelbe Suspension vor. Es wurde 30 Minuten nachgerührt. Die Reaktionsmischung wurde bei 5 bis 10 °C unter Rühren in eine Mischung aus 975 ml Eiswasser und 150 ml konzentrierter Schwefelsäure getropft. Die Reaktionsmischung wurde auf 40 bis 45 °C erwärmt. Die organische Phase wurde abgetrennt und einmal mit 250 ml Wasser gewaschen. Die organische Phase wurde eingeengt, wobei das Produkt in Form eines gelben Feststoffes anfiel.
Ausbeute: 194 g (96 %)
Regioselektivität (Methylgruppe in β-/α-Position; bestimmt mittels HPLC): 97:3

### Beispiel 2:

### Epoxidöffnung in Heptan bei -10 °C mit 1,3 Äquivalenten TMA bei inverser Fahrweise

97,5 ml (195 mmol) 2M Trimethylaluminiumlösung in Toluol wurden in 210 ml Heptan vorgelegt und bei -10 °C mit 38,1 g (150 mmol) 2,3-Epoxy-3,3-diphenylpropionsäuremethylester versetzt. Es wurde 30 Minuten nachgerührt. Die Reaktionsmischung wurde bei 5 bis 10 °C unter Rühren in eine Mischung aus 195 g Eiswasser und 30 ml konzentrierter Schwefelsäure getropft. Der Reaktor wurde mit 200 ml Toluol gewaschen. Die Reaktionsmischung wurde auf 40 bis 45 °C erwärmt. Die organische Phase wurde abgetrennt und die wässrige Phase einmal mit 200 ml Toluol nachextrahiert. Die vereinigten organischen Phasen wurden eingeengt, wobei das Produkt in Form eines gelben Feststoffes anfiel.
Ausbeute: 33,3 g (82 %)
Regioselektivität: 98:2

### Beispiel 3:

### Epoxidöffnung in Cyclohexan bei 5 °C mit 1,3 Äquivalenten TMA (6,25%ige Lösung)

5,0 g (19,7 mmol) 2,3-Epoxy-3,3-diphenylpropionsäuremethylester wurden in 80 ml Cyclohexan suspendiert. Bei 10 bis 15 °C wurden 15 ml (30 mmol) 2M Trimethylaluminiumlösung in Toluol zugegeben. Nach beendeter Zugabe lag eine klare gelbe Lösung vor. Es wurde 30 Minuten nachgerührt. Die Reaktionsmischung wurde bei 5 bis 10 °C unter Rühren in 250 ml 1M HCl-Lösung getropft. Es wurden weitere 60 ml Toluol zugegeben. Die organische Phase wurde abgetrennt und die wässrige Phase einmal mit Toluol nachextrahiert. Die vereinigten organischen Phasen wurden eingeengt, wobei das Produkt in Form eines gelben Feststoffes anfiel.
Ausbeute: 5,33 g (100 %)
Regioselektivität: 97:3

### Beispiel 4:

Beispiel 3 wurde wiederholt, wobei jedoch die Umsetzung in Cyclohexan bei 10 °C unter Verwendung von 1,3 Äquivalenten TMA (20%ige Lösung) erfolgte.
Ausbeute: 96 %
Regioselektivität: 90:10

### Beispiel 5:

Beispiel 3 wurde wiederholt, wobei jedoch die Umsetzung in Toluol bei -10 °C unter Verwendung von 1,3 Äquivalenten TMA erfolgte.
Ausbeute: 82 %
Regioselektivität: 91:9

### Beispiel 6:

Beispiel 3 wurde wiederholt, wobei jedoch die Umsetzung in Toluol bei 0 °C unter Verwendung von 1,5 Äquivalenten TMA erfolgte.
Ausbeute: 100 %
Regioselektivität: 90:10

### Beispiel 7:

Beispiel 3 wurde wiederholt, wobei jedoch die Umsetzung in Toluol bei 10 °C unter Verwendung von 1,5 Äquivalenten TMA erfolgte.
Ausbeute: 87 %
Regioselektivität: 78:22

### Beispiel 8:

Beispiel 3 wurde wiederholt, wobei jedoch die Umsetzung in Toluol bei 0 °C unter Verwendung von 2 Äquivalenten TMA erfolgte.
Ausbeute: 87 %
Regioselektivität: 86:14

## Patentansprüche

1. Verfahren zur Herstellung von 2-Hydroxycarbonsäureestern der Formel I 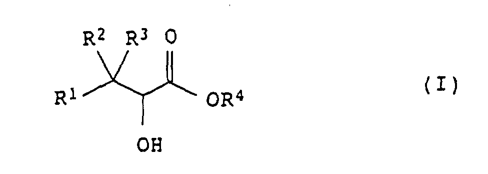 worin
R¹ und R² unabhängig voneinander für C₁-C₂₀-Alkyl, C₃-C₈-Cycloalkyl, C₂-C₂₀-Alkenyl, C₂-C₂₀-Alkinyl, C₆-C₁₀-Aryl oder C₇-C₁₄-Aralkyl oder C₇-C₂₀-Alkaryl stehen, oder R¹ und R² gemeinsam mit dem Kohlenstoffatom, an das sie gebunden sind, einen 5- bis 8-gliedrigen Ring bilden;
R³ für C₁-C₂₀-Alkyl, C₂-C₂₀-Alkenyl oder C₂-C₂₀-Alkinyl steht;
R⁴ für C₁-C₂₀-Alkyl, C₃-C₈-Cycloalkyl, C₂-C₂₀-Alkenyl, C₂-C₂₀-Alkinyl, C₆-C₁₀-Aryl, C₇-C₁₄-Aralkyl oder C₇-C₂₀-Alkaryl steht;
bei dem man einen Glycidester der Formel II 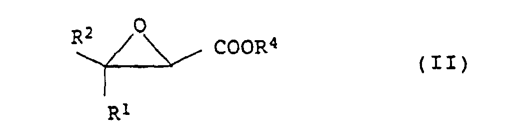 worin R¹, R² und R⁴ die angegebene Bedeutung haben,
mit einem aluminiumorganischen Reagenz der Formel III 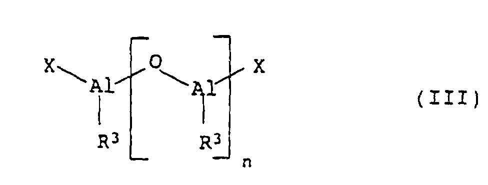 umsetzt, worin R³ die angegebenen Bedeutung hat, X jeweils unabhängig für die für R³ angegebenen Bedeutungen, Halogen oder C₁-C₄-Alkoxy steht und n für 0 bis 10 steht.

2. Verfahren nach Anspruch 1, wobei es sich bei dem aluminiumorganischen Reagenz um Trimethylaluminium handelt.

3. Verfahren nach Anspruch 1 oder 2, wobei R¹ und R² gleichzeitig für Phenyl stehen.

4. Verfahren nach einem der vorhergehenden Ansprüche, **dadurch gekennzeichnet, dass** die Umsetzung des Glycidesters der Formel II mit dem aluminiumorganischen Reagenz der Formel III bei einer Temperatur im Bereich von -10 bis +10 °C durchgeführt wird.

5. Verfahren nach einem der vorhergehenden Ansprüche, wobei die Umsetzung des Glycidesters der Formel II mit dem aluminiumorganischen Reagenz der Formel III in einem unter aliphatischen oder aromatischen Kohlenwasserstoffen ausgewählten Lösungsmittel durchgeführt wird.

6. Verfahren nach einem der vorhergehenden Ansprüche, wobei das molare Verhältnis des aluminiumorganischen Reagenzes der Formel III zum Glycidester der Formel II im Bereich von 1,3 bis 1,5 liegt.

7. Verfahren nach einem der vorhergehenden Ansprüche, wobei der Glycidester der Formel II zu einer Lösung des aluminiumorganischen Reagenzes der Formel III gegeben wird.

## Claims

1. A process for preparing 2-hydroxycarboxylic esters of the formula I 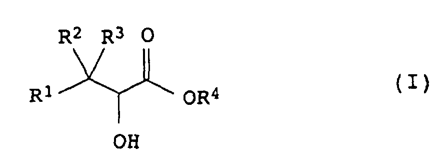 in which
R¹ and R² independently of one another are C₁-C₂₀-alkyl, C₃-C₈-cycloalkyl, C₂-C₂₀-alkenyl, C₂-C₂₀-alkynyl, C₆-C₁₀-aryl, C₇-C₁₄-aralkyl or C₇-C₂₀-alkylaryl, or R¹ and R² together with the carbon atom to which they are attached form a 5- to 8-membered ring;
R³ is C₁-C₂₀-alkyl, C₂-C₂₀-alkenyl or C₂-C₂₀-alkynyl;
R⁴ is C₁-C₂₀-alkyl, C₃-C₈-cycloalkyl, C₂-C₂₀-alkenyl, C₂-C₂₀-alkynyl, C₆-C₁₀-aryl, C₇-C₁₄-aralkyl or C₇-C₂₀-alkylaryl;
which comprises reacting a glycidyl ester of the formula II 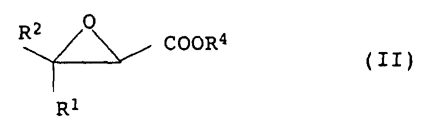 in which R¹, R² and R⁴ are as defined above
with an organoaluminum reagent of the formula III 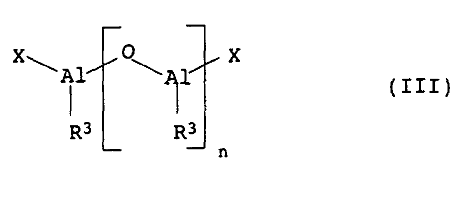 in which R³ is as defined above, X in each case independently have the meanings given for R³ or are halogen or C₁-C₄-alkoxy and n is from 0 to 10.

2. A process as claimed in claim 1, wherein the organoaluminum reagent is trimethylaluminum.

3. A process as claimed in claim 1 or 2, wherein R¹ and R² are both phenyl.

4. A process as claimed in any of the preceding claims, wherein the reaction of the glycidyl ester of the formula II with the organoaluminum reagent of the formula III is carried out at a temperature in the range from -10 to +10°C.

5. A process as claimed in any of the preceding claims, wherein the reaction of the glycidyl ester of the formula II with the organoaluminum reagent of the formula III is carried out in a solvent selected from the group consisting of aliphatic and aromatic hydrocarbons.

6. A process as claimed in any of the preceding claims, wherein the molar ratio of the organoaluminum reagent of the formula III to the glycidyl ester of the formula II is in the range from 1.3 to 1.5.

7. A process as claimed in any of the preceding claims, wherein the glycidyl ester of the formula II is added to a solution of the organoaluminum reagent of the formula III.

## Revendications

1. Procédé de préparation d'esters d'acides 2-hydrocarboxyliques répondant à la formule I 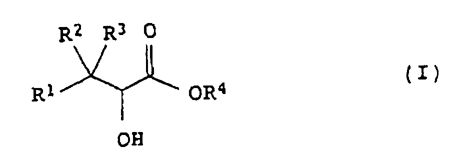 dans laquelle
R¹ et R² représentent, indépendamment l'un de l'autre, un groupe alkyle en C₁-C₂₀, cycloalkyle en C₃-C₈, alcényle en C₂-C₂₀, alcinyle en C₂-C₂₀, aryle en C₆-C₁₀, ou aralkyle en C₇-C₁₄, ou alcaryle en C₇-C₂₀, ou R¹ et R² forment ensemble avec l'atome de carbone auquel ils sont liés un cycle pentagonal à octogonal;
R³ représente un groupe alkyle en C₁-C₂₀, alcényle en C₂-C₂₀, alcinyle en C₂-C_{20;}
R⁴ représente un groupe alkyle en C₁-C₂₀, cycloalkyle en C₃-C₈, alcényle en C₂-C₂₀, alcinyle en C₂-C₂₀, aryle en C₆-C₁₀, aralkyle en C₇-C₁₄, ou alcaryle en C₇-C₂₀;
dans lequel on fait réagir un ester de glycide répondant à la formule II, 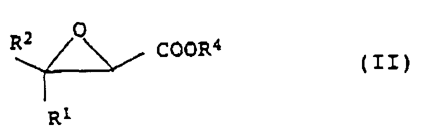 dans laquelle R¹, R² et R⁴ ont la signification indiquée, avec un réactif organoaluminium répondant à la formule III. 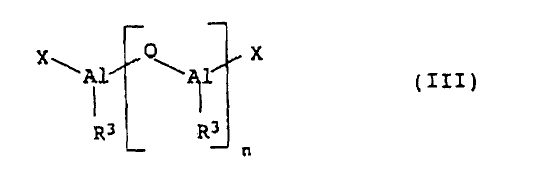 dans laquelle R³ a la signification indiquée, X représente à chaque fois, indépendamment les significations indiquées pour R³, un atome d'halogène ou un groupe alcoxy en C₁-C₄ et n a pour valeur 0 à 10.

2. Procédé selon la revendication 1, le réactif organoaluminium étant le triméthyl-aluminium.

3. Procédé selon la revendication 1 ou 2, R¹ et R² représentant en même temps le groupe phényle.

4. Procédé selon l'une quelconque des revendications précédentes, **caractérisé en ce que** la réaction de l'ester de glycide répondant à la formule II avec le réactif organoaluminium répondant à la formule III est effectuée à une température se situant dans une plage de -10 à + 10 °C.

5. Procédé selon l'une quelconque des revendications précédentes, **caractérisé en ce que** la réaction de l'ester de glycide répondant à la formule II avec le réactif organoaluminium répondant à la formule III est effectuée dans un solvant choisi parmi des hydrocarbures aliphatiques ou aromatiques.

6. Procédé selon l'une quelconque des revendications précédentes, le rapport molaire du réactif organoaluminium répondant à la formule III à l'ester de glycide répondant à la formule II se trouvant dans une plage de 1,3 à 1,5.

7. Procédé selon l'une quelconque des revendications précédentes, l'ester de glycide répondant à la formule II étant ajouté à une solution du réactif organoaluminium répondant à la formule III.
